# EUROPEAN PATENT APPLICATION

(11) **EP 4 458 940 A1**
(43) Date of publication of application: **06.11.2024**
(21) Application number: 22916672.3
(22) Date of filing: 26.12.2022
(51) Int. Cl.: C12J 1/04, A23L 2/38, A23L 33/105, C12G 3/02, C12G 3/026, C12R 1/865, C12R 1/02

(54) **METHOD FOR PREPARING FERMENTED HOVENIA DULCIS THUNB HAVING HANGOVER RELIEF AND LIVER PROTECTION EFFECTS, AND BEVERAGE COMPOSITION PREPARED USING SAME AS MAIN INGREDIENT**

(30) Priority: 28.12.2021 KR 20210189748
(71) Applicant: Morning Fighting International Co., Ltd., Seoul 06167 (KR)
(72) Inventor: SEO, Kwon Il, Busan 49329 (KR); PARK, Wool Lim, Busan 49315 (KR)
(74) Representative: Cabinet Chaillot
(86) International application number: PCT/KR2022/021334
(87) International publication number: WO 2023/128531

(57) **Abstract**

The present invention relates to: a method for preparing a Hovenia dulcis Thunb fruit fermented vinegar having hangover relief and liver protection effects; and a beverage composition prepared using the same as a main ingredient. In the method for preparing fermented vinegar by using Hovenia dulcis Thunb fruits of the present invention, a Hovenia dulcis Thunb fruits concentrate is made using Hovenia dulcis Thunb fruits and is adjusted to 16 °Brix. 10% (v/v) seed mash is inoculated thereto and subjected to alcohol fermentation at a temperature of 28 to 32 °C. Then obtained alcoholic fermentation filtrate is used as a substrate of acetic acid fermentation. In the acetic acid fermentation, a fermented liquid obtained through alcoholic fermentation of Hovenia dulcis Thunb fruits is inoculated with 10% (v/v) of a vinegar starter, and then fermented at a temperature of 28 to 32 °C under a 120 to 180 rpm condition.

## Description

### [Technical Field]

The present invention relates to a method for preparing fermented Hovenia dulcis Thunb having hangover relief and liver protection effects, and a beverage composition prepared using the same as a main ingredient. More specifically, it relates to preparing fermented vinegar having hangover relief and liver protection effects by fermenting Hovenia dulcis Thunb or Hovenia dulcis Thunb fruits, and to preparing a beverage composition using the same.

### [Background Art]

Hovenia dulcis Thunb is a deciduous broadleaf tree belonging to the Rhamnaceae family, and also known as hokkae tree, geomancy tree, and white stone tree. In Oriental medicine, it is known to be excellent for improving liver function and detoxifying alcohol.

In plant illustration and herbal medicine, the fruit of Hovenia dulcis Thunb is known to have excellent diuretic, thirst-relieving, liver function-protecting, and alcohol poisoning-removing properties. Recently, the pharmacological effects of Hovenia dulcis Thunb fruit, such as anti-diabetic, antioxidant, promoting alcohol metabolism, eliminating hangovers, and relieving thirst, have been known, and efforts are being made in various ways to use it in the production of health functional foods.

Moderate consumption of alcohol in the human body helps promote health and social interaction, but excessive drinking causes hangovers such as vomiting, thirst, muscle pain, headache, and nausea, which interfere with daily life. In addition, continuous alcohol consumption causes metabolic disorders and impaired nutrient absorption in the body, which leads to malnutrition, alcoholic fatty liver disease, cirrhosis, hepatitis, and other liver diseases, as well as diabetes, neuropsychiatric disorders, and cancer.

Alcohol is mainly metabolized in the liver to form acetaldehyde and then oxidized to acetate. Liver cells are damaged by acetaldehyde, the substance that causes hangovers in the body, and excessive alcohol affects lipid metabolism, causing excessive production of fatty acids and decreased fatty acid oxidation, which induces fatty liver. Therefore, research is being conducted to eliminate hangovers and suppress liver damage caused by alcohol consumption.

Recently, due to changes in disease patterns, consumers' interest in slow food, such as healthy foods such as fermented foods and traditional foods, is increasing, and interest in hangover relief drinks using Hovenia dulcis is also increasing.

Vinegar is a representative fermented food with a long history in the human diet along with alcohol, and contains sourness, sugars, volatile or nonvolatile organic acids, esters, amino acids, etc. The components of vinegar are reported to have physiological functions such as recovery from fatigue and prevention of aging, and effects such as hypertension, arteriosclerosis, and regulation of body metabolism, and are attracting attention as a health functional food. Vinegar is classified into brewed vinegar made with fruits, grains, etc. as the main ingredient, and synthetic vinegar made by diluting acetic acid or glacial acetic acid with water.

Recently, as the food culture has improved and awareness of health has increased, research on natural brewed vinegar using various fruits and vegetables such as apples, persimmons, and grapes has been actively conducted rather than synthetic vinegar. However, research on vinegar using Hovenia dulcis Thunb fruit for hangover relief and liver protection has been insufficient.

### [Disclosure]

### [Technical Problem]

The present invention is intended to solve the above-mentioned problems, and its purpose is to materialize the acetic acid fermentation concentrate containing Hovenia dulcis Thunb fruits as the main ingredient, which is reported to be excellent in improving liver function and decomposing alcohol, into a functional food, and to provide a functional beverage having hangover relief and liver function protection effects.

### [Technical Solution]

In order to solve the above technical problem, the present invention prepares fermented vinegar using Hovenia dulcis Thunb fruits and prepares a functional beverage having hangover relief and liver protection effects.

The method for preparing fermented vinegar by using Hovenia dulcis Thunb fruits of the present invention uses Hovenia dulcis Thunb fruits to prepare Hovenia dulcis Thunb fruits concentrate, adjusts the concentrate to 16°Brix, inoculates 10% (v/v) of seed mash therein, performs alcohol fermentation at a temperature of 28 to 32°C, and then uses the filtered alcohol fermentation filtrate as a substrate for acetic acid fermentation. The acetic acid fermentation is performed by inoculating 10% (v/v) of vinegar starter into the fermented liquid obtained through the alcoholic fermentation of Hovenia dulcis Thunb fruits, and then fermenting under the conditions of 120 to 180 rpm at a temperature of 28 to 32°C.

The Hovenia dulcis Thunb fruits concentrate can be prepared by adding water to washed Hovenia dulcis Thunb fruits in an amount 5 to 10 times the weight of the fruits, heating them, concentrating them through hot water extraction, or by alcohol extraction.

The alcohol fermentation is performed by subculturing the strain Saccharomyces cerevisiae (KCCM 11306) on YM plate medium and then culturing it in YM liquid medium for 48 hours.

The YM plate medium was prepared by diluting the above fermentation strain with a 0.85% NaCl solution, spreading it on LB, YM, PDA, and MRS plate medium, and then culturing it in an incubator at 28 to 32°C and then culturing it in a liquid medium.

The YM liquid medium uses a medium containing 0.5 to 1.5 parts by weight of Dextrose, 0.2 to 0.8 parts by weight of Peptone, 0.1 to 0.5 parts by weight of Yeast extract, and 0.1 to 0.5 parts by weight of Malt extract in 100 parts by weight of water.

The acetic acid fermentation is performed by culturing the strain Acetobacter aceti (KCCM 12654) at 30°C for 48 hours in a liquid medium containing 2 to 3 parts by weight of mannitol, 0.1 to 0.5 parts by weight of peptone, and 0.3 to 0.8 parts by weight of yeast extract in 100 parts by weight of water.

The beverage composition using Hovenia dulcis Thunb fruit fermented vinegar of the present invention is prepared by including 0.5 to 7 parts by weight of Hovenia dulcis Thunb fruit fermented vinegar, 1 to 3 parts by weight of Hovenia dulcis concentrate, 10 to 15 parts by weight of high fructose corn syrup, 1 to 5 parts by weight of fructooligosaccharide, 0.5 to 1 parts by weight of apple concentrate, 0.1 to 0.2 parts by weight of anhydrous citric acid, 0.1 parts by weight of yeast extract powder, 0.01 to 0.03 parts by weight of Complex Scutellaria baicalensis extract, 1 to 2 parts by weight of taurine, 0.01 to 0.10 parts by weight of milk thistle extract powder, and 0.01 to 0.05 parts by weight of curcumin in 100 parts by weight of purified water.

### [Advantageous Effects]

The present invention has the effect of providing fermented vinegar using Hovenia dulcis Thunb fruits, which are excellent in improving liver function and decomposing alcohol.

In addition, the present invention can provide a hangover relief beverage by materializing an acetic acid fermentation concentrate having the fermented vinegar as a main ingredient into a functional food, and has the effect of providing a functional beverage having a liver function protection effect.

### [Description of Drawings]

In the method for preparing fermented vinegar by using Hovenia dulcis Thunb fruits of the present invention, Hovenia dulcis Thunb fruits concentrate is made using Hovenia dulcis Thunb fruits, and after adjusting it to 16°Brix, 10% (v/v) seed mash is inoculated, alcohol fermentation is performed at a temperature of 28 to 32°C, and the filtered alcohol fermentation filtrate is used as a substrate for acetic acid fermentation. The acetic acid fermentation is performed by inoculating 10% (v/v) of vinegar starter into the fermented liquid obtained through the alcoholic fermentation of Hovenia dulcis Thunb fruits, and then fermenting under the conditions of 120 to 180 rpm at a temperature of 28 to 32°C.

### [Best Model]

Hereinafter, in order to help understand the present invention, Examples, etc. will be described in detail. However, Examples according to the present invention may be modified in various different forms, and the scope of the present invention should not be construed as being limited to the Examples below. Examples of the present invention are provided to more completely explain the present invention to a person having average knowledge in the art.

As used throughout the specification of the present invention, the term 'fermented Hovenia dulcis' is defined to mean a case where the inventors prepare 'fermented vinegar, by fermenting Hovenia dulcis Thunb or Hovenia dulcis Thunb fruits, and more precisely, fermented Hovenia dulcis means 'Hovenia dulcis Thunb fruit fermented vinegar'.

In the method for preparing fermented vinegar by using Hovenia dulcis Thunb fruits of the present invention, Hovenia dulcis Thunb fruits concentrate is made using Hovenia dulcis Thunb fruits, and after adjusting it to 16°Brix, 10% (v/v) seed mash is inoculated, alcohol fermentation is performed at a temperature of 28 to 32°C, and the filtered alcohol fermentation filtrate is used as a substrate for acetic acid fermentation. When controlling the sugar content of the Hovenia dulcis Thunb fruits concentrate, it is controlled with a saccharifying solution and adjusted to 14 to 18°Brix, preferably 16°Brix.

The seed mash is prepared by inoculating the alcohol fermentation strain Saccharomyces cerevisiae (KCCM 11306) into Hovenia dulcis Thunb fruits concentrate adjusted to 14 to 16°Brix and cultivating it at a temperature of 28 to 32°C for 40 to 56 hours, and using it as seed mash.

The acetic acid fermentation is performed by inoculating 10% (v/v) of vinegar starter into the fermented liquid obtained through the alcoholic fermentation of Hovenia dulcis Thunb fruits, and then fermenting under the conditions of 120 to 180 rpm at a temperature of 28 to 32°C. The vinegar starter was prepared by filtering the Hovenia dulcis Thunb fruits alcohol fermentation liquid obtained from alcohol fermentation, inoculating the filtrate with the acetic acid bacteria Acetobacter aceti (KCCM deposited strain: 12654), and culturing it for 48 hours while shaking it at a temperature of 28 to 32°C and a rotation speed of 120 to 180 rpm, preferably 150 rpm, and using it as a vinegar starter.

The Hovenia dulcis Thunb fruits concentrate can be manufactured by adding water to washed Hovenia dulcis Thunb fruits in an amount 5 to 10 times the weight of the fruits, heating them, concentrating them through hot water extraction, or by alcohol extraction.

The alcohol fermentation is performed by subculturing the strain Saccharomyces cerevisiae (KCCM 11306) on YM plate medium and then culturing it in YM liquid medium for 48 hours.

The YM plate medium was prepared by diluting the above fermentation strain with a 0.85% NaCl solution, spreading it on LB, YM, PDA, and MRS plate medium, and then culturing it in an incubator at 28 to 32°C and then culturing it in a liquid medium.

The YM liquid medium uses a medium containing 0.5 to 1.5 parts by weight of Dextrose, 0.2 to 0.8 parts by weight of Peptone, 0.1 to 0.5 parts by weight of Yeast extract, and 0.1 to 0.5 parts by weight of Malt extract in 100 parts by weight of water.

The acetic acid fermentation is performed by culturing the strain Acetobacter aceti (KCCM 12654) at 30°C for 48 hours in a liquid medium containing 2 to 3 parts by weight of mannitol, 0.1 to 0.5 parts by weight of peptone, and 0.3 to 0.8 parts by weight of yeast extract in 100 parts by weight of water.

The beverage composition using Hovenia dulcis Thunb fruit fermented vinegar of the present invention is manufactured by including 0.5 to 7 parts by weight of Hovenia dulcis Thunb fruit fermented vinegar, 1 to 3 parts by weight of Hovenia dulcis concentrate, 10 to 15 parts by weight of high fructose corn syrup, 1 to 5 parts by weight of fructooligosaccharide, 0.5 to 1 parts by weight of apple concentrate, 0.1 to 0.2 parts by weight of anhydrous citric acid, 0.1 parts by weight of yeast extract powder, 0.01 to 0.03 parts by weight of Complex Scutellaria baicalensis extract, 1 to 2 parts by weight of taurine, 0.01 to 0.10 parts by weight of milk thistle extract powder, and 0.01 to 0.05 parts by weight of curcumin in 100 parts by weight of purified water.

Silymarin contained in the milk thistle (Silybum marianum L. Gaertner) is a flavanolignan and actually includes a mixture of four isomers, silybin, isosilybin, silydianin, and silychristin, which have the same molecular formula of C25H22O10.

The above silymarin has been reported to inhibit the proliferation of cancer cells and to be effective against diabetes, and also has antimicrobial activity against gram-positive bacteria. It has also been recently reported to be effective against skin damage caused by radiation and ultraviolet rays, and has been reported to have a liver-protecting effect, preventing alcohol-induced lipid oxidation, and protecting effect against alcoholic cirrhosis.

Curcumin is a type of polyphenol mainly found in Indian turmeric. Curcumin is known to give turmeric its yellow color.

The curcumin is a food additive used to color food with a slightly yellow color and has an E code of E100. Curcumin and a specific component related to curcumin, PPAR-gamma (Peroxisome Proliferator Activated Receptor-gamma), act as a mechanism to block mediators that cause inflammation, and have anti-inflammatory, antioxidant, and antibacterial effects.

In addition, ingredients including yeast extract powder and Complex Scutellaria baicalensis extract are purchased commercially and mixed for use.

### 1. Acute alcohol administration animal experiment design

In the present invention, the experimental animals were 48 male Sprague-Dawley rats obtained from Hyochang Science (Daegu, Korea). The animal rearing room was maintained with a constant temperature (22 ± 2°C) and humidity (50 ± 5%) and a 12-hour light/dark cycle. According to randomized block design, the animals were divided into alcohol-only administration control group for acute alcohol dissolution (Control), 1.5% Hovenia dulcis Thunb fruits concentrate group (HT), Hovenia dulcis Thunb fruit fermented vinegar 4% diluted solution group (PHT), Hovenia dulcis hangover drink group (DHD), and positive control group (A, B).

In the case of the positive control group, a third-party product sold as a hangover drink on the market was used. Animals in each group were separated into two acrylic cages and used after acclimating to solid diet for one week. On the day of the experiment, each sample was administered to the rats at 7 ml/kg after fasting for 15 hours. 30 minutes later, 5 ml/kg of 40% fermented alcohol(ethanol) was administered orally, and blood was collected 180 minutes later to measure the concentrations of alcohol and acetaldehyde. The collected blood was left at room temperature for 3 hours, centrifuged at 12000 rpm for 10 minutes, and only the supernatant was taken and used in the experiment.

### 2. Measurement of blood alcohol and acetaldehyde concentrations

Alcohol concentration in serum was measured using an ethanol analysis kit (Megazyme, Ireland), and acetaldehyde concentration in serum was measured using an acetaldehyde analysis kit (Megazyme, Ireland).

### 3. Experiment result

### A) Measurement of alcohol and acetaldehyde concentrations in an acute alcoholic rat model

The alcohol dissolution results of the acute alcoholic rat model are as follows. Alcohol and acetaldehyde concentrations were highest in the Control group, and similar concentrations were found in the HT group. The PHT group showed a significant decrease in blood alcohol and acetaldehyde concentrations compared to the Control and HT groups. The DHD group was confirmed to have decreased more than the positive control group A and B, and in particular, there was a significant difference from group A. Consumption of Hovenia dulcis Thunb fruit fermented vinegar beverage is considered to have an excellent ability to decompose alcohol in the body as it reduces the concentration of alcohol and acetaldehyde in the blood.

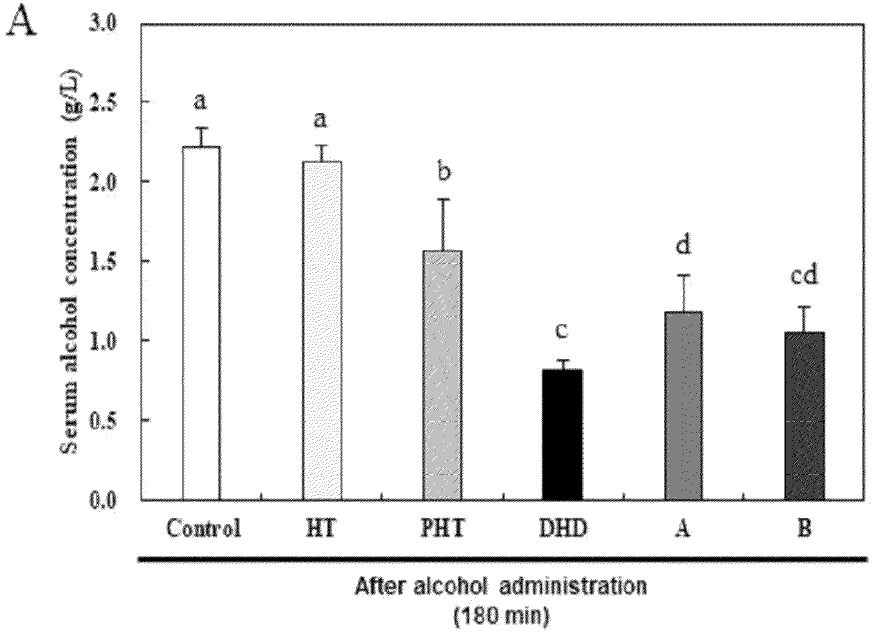

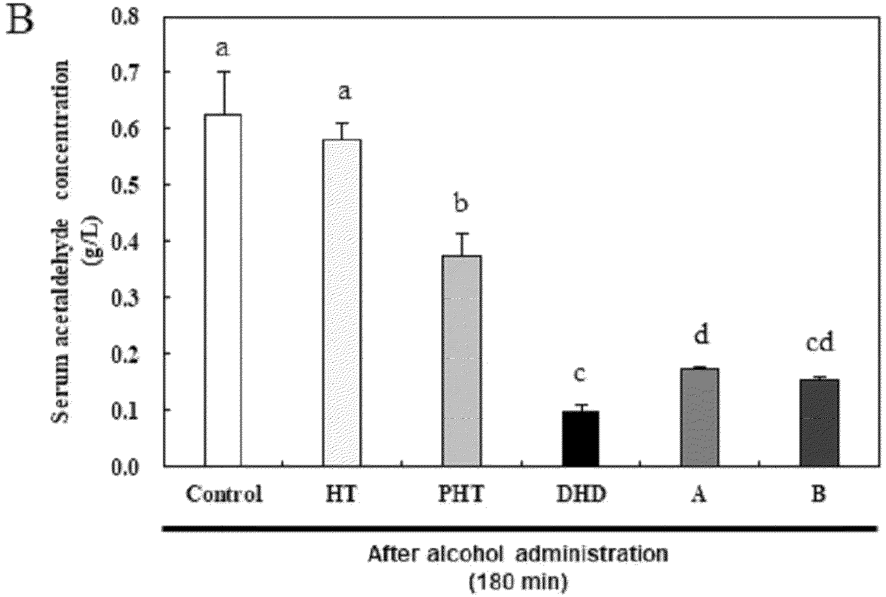

**Fig. Effects of DHD on (A) alcohol and (B) acetaldehyde concentration in serum.** Data values with different superscripts indicate significant difference (p 003c# 0.05) by Duncan's multiple range tests.

4. Confirmation of hangover relief and liver protection effects of Hovenia dulcis Thunb fruit fermented vinegar beverage containing Hovenia dulcis Thunb fruits as main ingredient in chronic alcohol administration model

### I. Materials and Methods

### A) Chronic alcohol administration animal experiment design

The experimental animals were 48 male Sprague-Dawley rats obtained from Hyochang Science (Daegu, Korea), and divided into a total of 6 groups: Normal group (Normal), alcohol-only administration control group (Control), 1.50 Hovenia dulcis Thunb fruits concentrate group (HT), processed Hovenia dulcis Thunb fruit 4% diluted solution group (PHT), Hovenia dulcis hangover drink group (DHD), and positive control group (Silymarin) administered silymarin, a known liver function improver. Animals in each group were separated into two acrylic cages according to a randomized block design, adapted to a solid diet for one week, and then tested. To prevent acute toxicity caused by alcohol, 10 and 20% fermented alcohol were administered for one week each, and each sample was administered at the same time as 30% fermented alcohol for 4 weeks. The sample was administered at 50 mg/kg to the positive control group, 7 ml/kg to the remaining groups except the positive control group, and the same amount of distilled water was administered to the normal group and control group. On the day of the experiment, they were fasted for 15 hours and then sacrificed to collect blood and organs. The collected blood was left at room temperature for 3 hours, centrifuged at 12000 rpm for 10 minutes, and only the supernatant was taken and used in the experiment.

The basic diet used in this experiment was a solid diet. Water, alcohol, and diet were provided freely, and body weight, food intake, and water or alcohol intake were measured every day. Animal experiments were conducted ethically in accordance with Dong-A University's standards for use of laboratory animals.

### B) Measurement of blood alcohol and acetaldehyde concentrations

Alcohol concentration in serum was measured using an ethanol analysis kit (Megazyme, Ireland), and acetaldehyde concentration in serum was measured using an acetaldehyde analysis kit (Megazyme, Ireland).

### C) Measurement of alcohol dehydrogenase (ADH) and acetaldehyde dehydrogenase (ALDH) activity in liver tissue

100 mg of liver tissue was homogenized by mixing with 500 ul of 1xPBS and then centrifuged at 10000 rpm, 4°C for 5 minutes, and the supernatant was collected and used in the experiment. ADH activity was measured using an ADH activity kit (Bioassay systems, San Francisco Bay), and ALDH activity was measured using an ALDH assay kit (BioVision, California).

### D) Measurement of lipoperoxide content

Lipoperoxide was measured by the content of thiobarbituric acid reactive substance (TBARS). 1 ml of a solution prepared by homogenizing liver tissue and 2 ml of TBA (2-thiobarbituric acid) reagent were stirred and reacted in boiling water for 30 minutes. Afterwards, the reaction was stopped with ice water, centrifuged at 3000 rpm for 10 minutes, the supernatant was taken, and the absorbance was measured at 535 nm.

### E) Analysis of lipid concentration and biochemical indicator substances

Serum LDL and HDL-cholesterol, triglyceride content, ALT (GPT), AST (GOT), and GGT (γ-GTP) concentrations were analyzed at Southeast Medi-Chem Institute (Korea), a specialized analysis institute. Total cholesterol content in blood and triglyceride concentration in liver tissue were measured using an analysis kit (Elabscience, United States).

### F) Glutathione content analysis

Gluthione content in serum and liver tissue was analyzed using a kit (Elabscience, United States).

### G) Hematoxylin and Eosin (H0026#E) staining analysis

Liver tissue was fixed with 4% fromaldehyde solution, stained with H0026#E solution, and observed under an optical microscope.

### II. Experiment result

### A) Changes in body weight and beverage intake in a chronic alcoholic rat model

The results of changes in body weight and beverage intake of chronic ethanol-administered animals are shown in the table below. The initial body weight of the rats showed no significant difference in all groups, but the final body weight showed a significant difference between the Control and Normal groups. The HT group, PHT group, and Silymarin group showed no significant difference from the Control group, but body weight increased in rats in the PHT group. In the case of the DHD group, there was no significant difference from the Normal group. There was no significant difference in beverage intake among all groups.

### Table. Effect of DHD on body weight, weight gain and water consumption in chronic ethanol-administered rats

| | **Normal** | **Control** | **HT** | **PHT** | **DHD** | **Silymarin** |
|---|---|---|---|---|---|---|
| **Body weight (g)** | | | | | | |
| **Initial** | **254.50 ± 5.36^{a}** | **253.75 ± 13.74^{a}** | **254.17 ± 15.87^{a}** | **254.50 ± 8.52^{a}** | **254.83 ± 2.93^{a}** | **253.67 ± 17.76^{a}** |
| **Final** | **434.50 ± 32.40^{a}** | **353.50 ± 24.25^{b}** | **373.33 ± 12.91^{bc}** | **387.67 ± 9.29^{bc}** | **409.33 ± 4.04^{ac}** | **372.33 ± 7.09^{bc}** |
| **Weight gain** | **177.42 ± 32.00^{a}** | **97.50 ± 29.07^{b}** | **127.33 ± 9.52^{bc}** | **137.33 ± 18.33^{c}** | **154.50 ± 4.92^{ac}** | **118.67 ± 10.91^{bc}** |
| **Water consumption (ml/day)** | **53.98 ± 11.34^{a}** | **42.16 ± 12.20^{a}** | **42.63 ± 11.94^{a}** | **41.54 ± 11.28^{a}** | **42.70 ± 9.62^{a}** | **42.92 ± 11.88^{a}** |

Data values were expressed as mean ± S.E. (n=6). Data values with different superscripts indicate significant difference (p 003c# 0.05) by Duncan's multiple range tests.

### B) Organ weight changes in a chronic alcoholic rat model

Organ weight changes in chronic ethanol-administered rats are shown in the table below. There was no significant difference in the weight of the spleen, kidney, and heart in all groups. The weight of the liver was found to be highest in the Control group, while the lowest weight was found in the Normal group. It was confirmed that the liver weight of the HT group, PHT group, and Silymarin group was significantly decreased compared to the Control group, and the PHT group was confirmed to have a lower liver weight than the HT group and Silymarin group. The DHD group showed lower weight than the HT group, PHT group, and Silymarin group, and there was no significant difference from the Normal group. In the case of testis weight, the Control group showed the highest weight, and the PHT group showed lower weight than the HT and Silymarin groups. The DHD group showed lower testis weight with no significant difference from the Normal group. Consumption of Hovenia dulcis Thunb fruit fermented vinegar beverage is believed to suppress alcoholic fatty liver disease.

### Table. Effect of DHD on organ weights in chronic ethanol-administered rats

| | **Normal** | **Control** | **HT** | **PHT** | **DHD** | **Silymarin** |
|---|---|---|---|---|---|---|
| **Liver (g)** | **9.03 ± 0.22^{a}** | **12.83 ± 0.70^{b}** | **11.43 ± 0.95^{c}** | **10.39 ± 0.27^{c}** | **9.26 ± 0.21^{a}** | **10.93 ± 0.27^{c}** |
| **Spleen (g)** | **0.73 ± 0.07^{a}** | **0.69 ± 0.09^{a}** | **0.69 ± 0.07^{a}** | **0.71 ± 0.07^{a}** | **0.72 ± 0.06^{a}** | **0.69 ± 0.01^{a}** |
| **Kidney (g)** | **2.40 ± 0.11^{a}** | **2.63 ± 0.04^{a}** | **2.61 ± 0.19^{a}** | **2.55 ± 0.11^{a}** | **1.41 ± 0.07^{a}** | **2.61 ± 0.06^{a}** |
| **Testis (g)** | **3.36 ± 0.14^{a}** | **3.94 ± 0.19^{b}** | **3.81 ± 0.15^{b}** | **3.67 ± 0.17^{ab}** | **3.40 ± 0.26^{a}** | **3.78 ± 0.19^{b}** |
| **Heart (g)** | **1.17 ± 0.02^{a}** | **1.09 ± 0.13^{a}** | **1.10 ± 0.08^{a}** | **1.11 ± 0.05^{a}** | **1.15 ± 0.03^{a}** | **1.08 ± 0.11^{a}** |

Data values were expressed as mean ± S.E. (n=6). Data values with different superscripts indicate significant difference (p 003c# 0.05) by Duncan's multiple range tests.

### B) Changes in liver damage markers in a chronic alcoholic rat model

The results of measuring liver damage indicators in chronic ethanol-administered rats are shown in the figure below. GOT, GPT, and γ-GTP concentrations were found to increase the most in the Control group and the lowest in the Normal group. GOT, GPT, and γ-GTP concentrations were found to be significantly decreased in the PHT group compared to the Control group, HT group, and Silymarin group. The concentrations of GOT, GPT, and γ-GTP in the DHD group showed no significant difference from the Normal group, and were significantly lower than those in the Control group, HT group, PHT group, and Silymarin group. Consumption of Hovenia dulcis Thunb fruit fermented vinegar beverage is believed to protect the liver from alcohol-related liver damage.

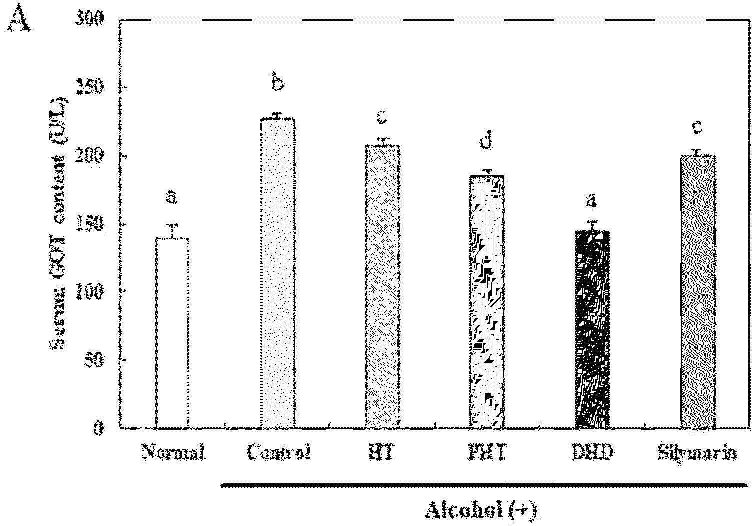

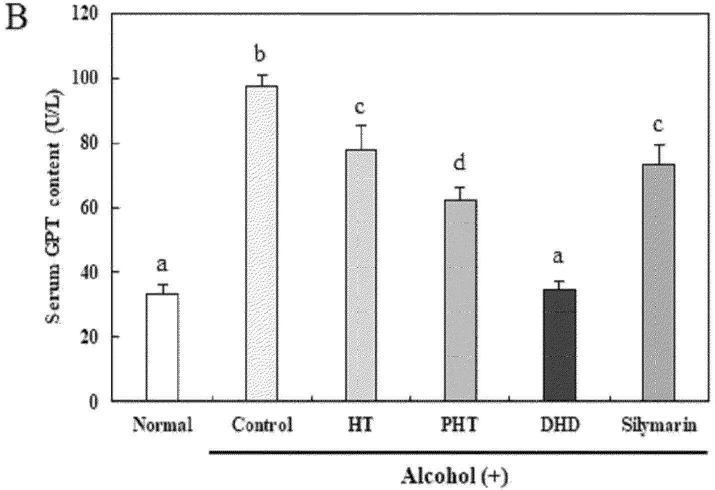

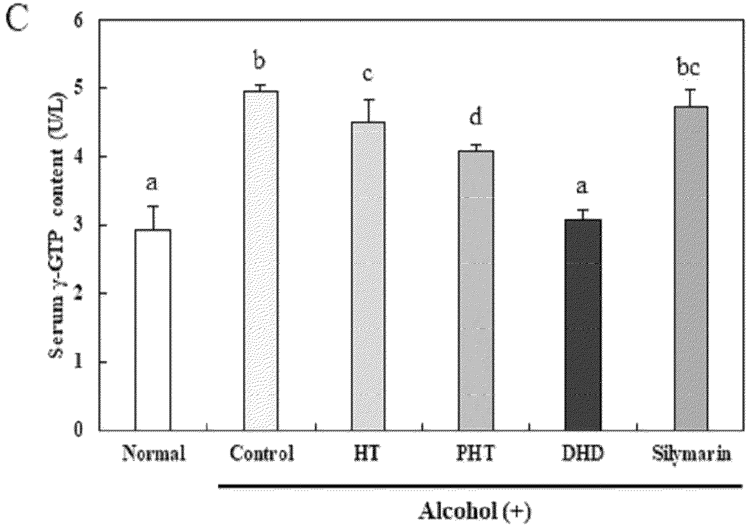

**Fig. Effects of DHD on (A) GOT (AST) content and (B) GPT(ALT) content in (C) γ-GTP content serum in chronic ethanol-administered rats.** Data values were expressed as mean ± S.E. (n=6). Data values with different superscripts indicate significant difference (p 003c# 0.05) by Duncan's multiple range tests.

### C) Changes in alcohol dehydrogenase activity (ADH, ALDH) in a chronic alcoholic rat model

The results of ADH and ALDH activities in chronic ethanol-administered rats are as follows. ADH activity was higher in the HT group, PHT group, DHD group, and Silymarin group than in the Control group. In particular, the Silymarin group, PHT group, and DHD group showed a significant increase. ADH activity significantly increased in the PHT group compared to the HT and Silymarin groups, and was significantly highest in the DHD group. ALDH activity in the PHT group was higher than the Control group, HT group, and Silymarin group, and was especially significantly higher than the Silymarin group. The DHD group showed significantly higher ALDH activity. Consumption of Hovenia dulcis Thunb fruit fermented vinegar beverage is believed to increase alcohol decomposition enzyme activity.

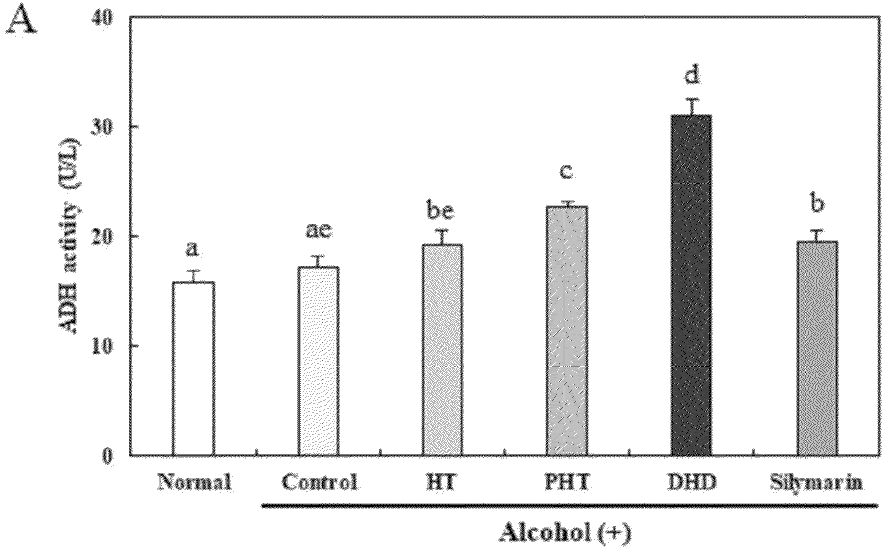

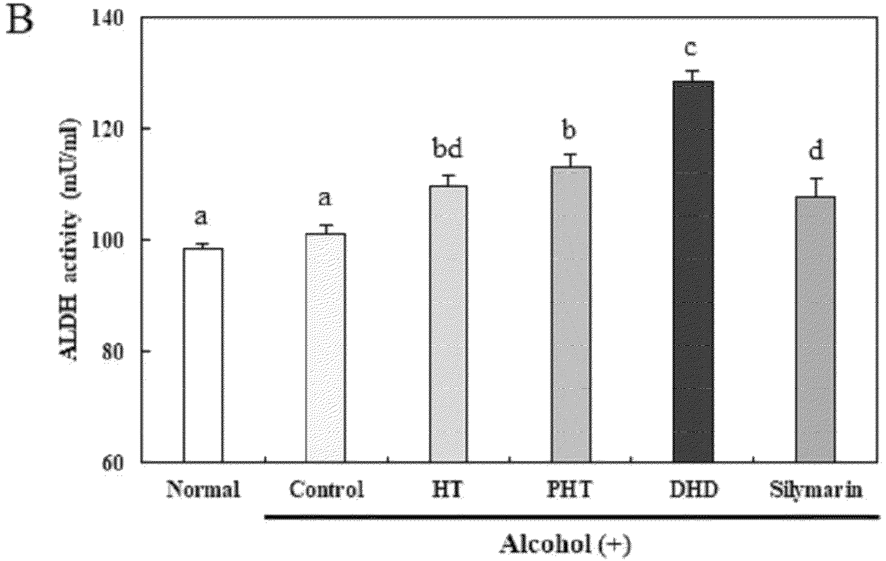

**Fig. Effects of DHD on (A) ADH activity and (B) ALDH activity in liver in chronic ethanol-administered rats.** Data values were expressed as mean ± S.E. (n=6). Data values with different superscripts indicate significant difference (p 003c# 0.05) by Duncan's multiple range tests.

### D) Changes in blood alcohol and acetaldehyde concentrations in a chronic alcoholic rat model

The results of alcohol and acetaldehyde concentrations in chronic ethanol-administered rats are shown in the figure below. Blood alcohol concentration was found to be significantly highest in the Control group and lowest in the Normal group. The PHT group showed a significantly lower concentration than the HT group, and the DHD group showed a significantly lower concentration than the HT, PHT, and Silymarin groups, and showed no significant difference from the Normal group. Acetaldehyde concentration was found to be significantly highest in the Control group and lowest in the Normal group. The PHT group showed a significantly lower acetaldehyde concentration than the HT group and Silymarin group, and the DHD group showed a significantly lower acetaldehyde concentration than the HT group, PHT group, and Silymarin group. Additionally, the DHD group showed no significant difference from the Normal group. Consumption of Hovenia dulcis Thunb fruit fermented vinegar beverage is believed to reduce alcohol and acetaldehyde concentrations in the blood.

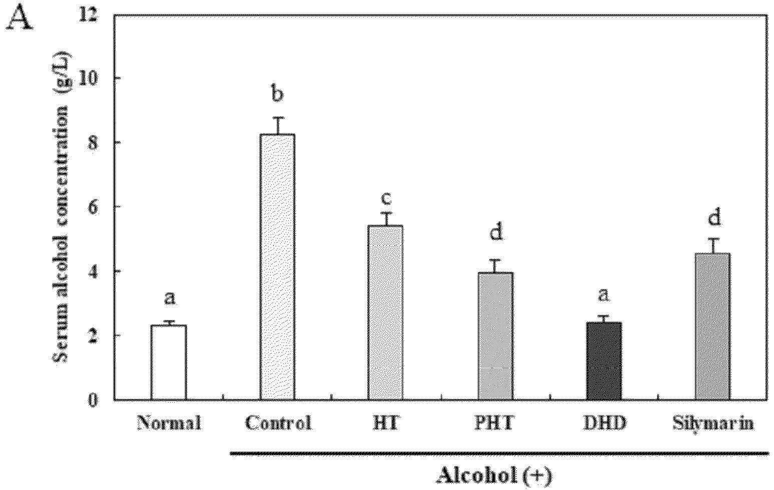

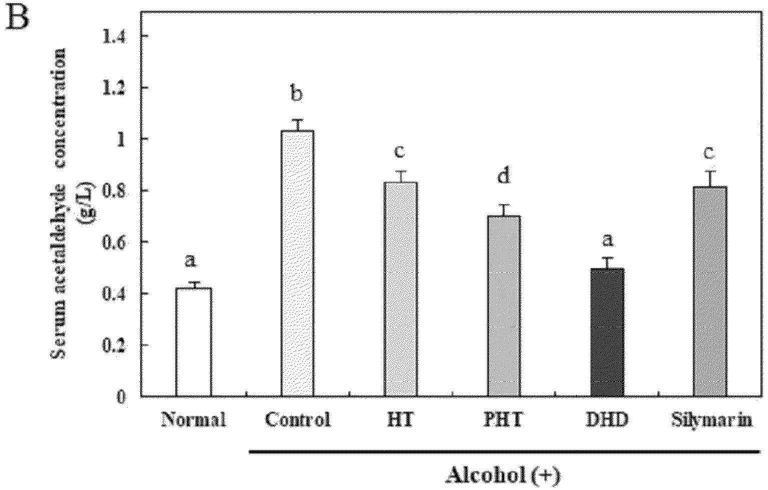

**Fig. Effects of DHD on (A) alcohol and (B) acetaldehyde concentration in serum in chronic ethanol-administered rats.** Data values were expressed as mean ± S.E. (n=6). Data values with different superscripts indicate significant difference (p 003c# 0.05) by Duncan's multiple range tests.

### E) Changes in liver and blood glutathione (GSH) concentrations in a chronic alcoholic rat model

The measurement results of liver and blood GSH concentrations in chronic ethanol-administered rats are shown in the figure below. Liver tissue and blood GSH concentrations were found to be lowest in the Control group. The concentration of the HT group was higher than that of the Control group, but there was no significant difference, and the PHT group showed a higher concentration than the HT group, although there was no significant difference. The DHD group showed significantly higher liver and blood GSH concentrations. Consumption of Hovenia dulcis Thunb fruit fermented vinegar beverage is believed to activate GSH in the liver and blood.

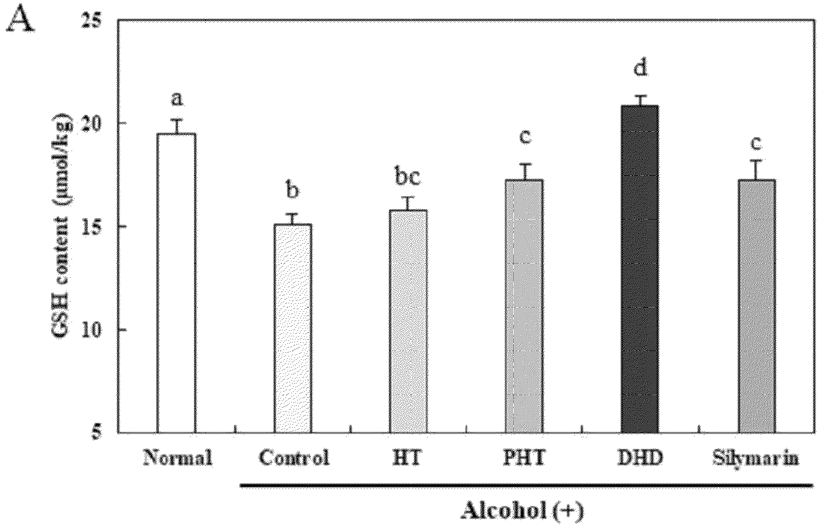

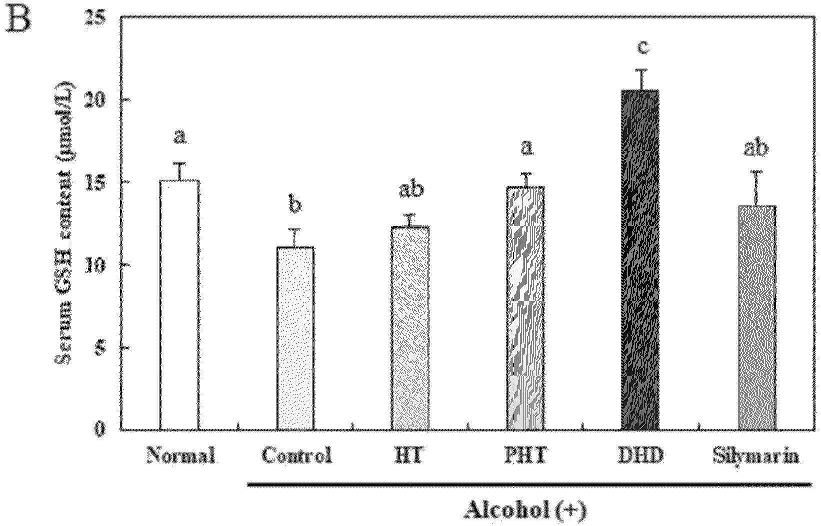

**Fig. Effects of DHD on (A) GSH content in liver and (B) GSH content in serum in chronic ethanol-administered rats.** Data values were expressed as mean ± S.E. (n=6). Data values with different superscripts indicate significant difference (p 003c# 0.05) by Duncan's multiple range tests.

### F) Changes in liver and blood TBARS (MDA) concentrations in a chronic alcoholic rat model

Liver and blood lipoperoxide concentrations in chronic alcohol rats are shown in the figure below. Lipoperoxide concentration in the liver was highest in the Control group and lowest in the Normal group. The HT group had a lower concentration of lipoperoxide than the Control group, but there was no significant difference, and the PHT group had a significantly lower concentration than the HT group and a lower concentration than the Silymarin group. The DHD group showed significantly lower lipoperoxide concentration than the PHT group and showed no significant difference from the Normal group. In the case of lipoperoxide in the blood, the control group showed significantly higher concentration. The HT group and Silymarin group were found to have significantly lower concentrations than the Control group, and the PHT group showed significantly lower concentrations than the HT and Silymarin groups. The DHD group showed a significantly lower concentration than the PHT group and showed no significant difference from the Normal group. Consumption of Hovenia dulcis Thunb fruit fermented vinegar beverage is believed to inhibit lipoperoxide production in the liver and blood.

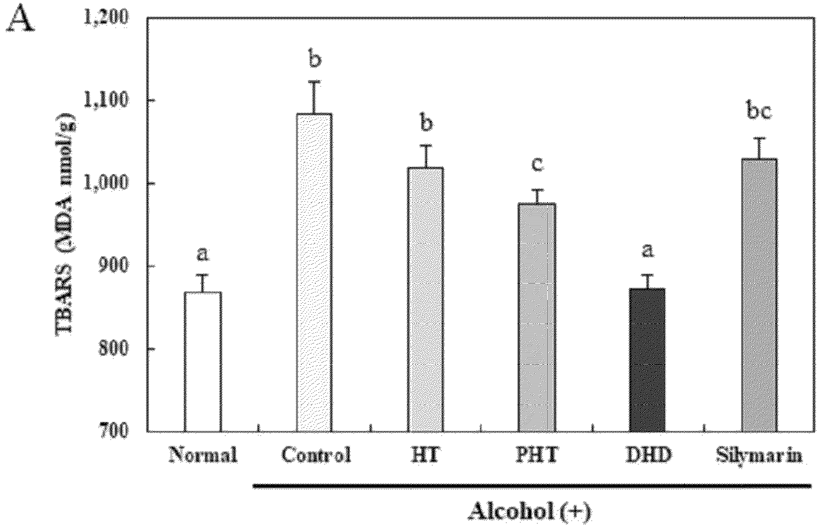

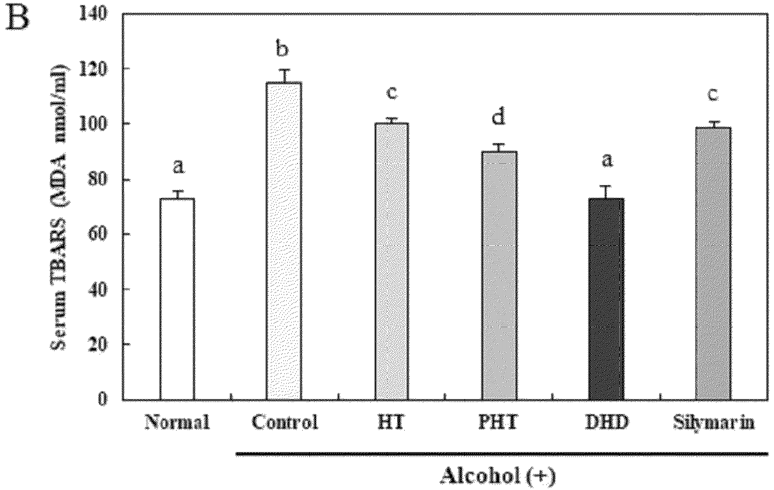

**Fig. Effects of DHD on (A) TBARS concentration in liver and (B) TBARS concentration in serum in chronic ethanol-administered rats.** Data values were expressed as mean ± S.E. (n=6). Data values with different superscripts indicate significant difference (p 003c# 0.05) by Duncan's multiple range tests.

### G) Changes in liver and blood triglyceride concentrations in a chronic alcoholic rat model

The measurement results of liver and blood triglyceride concentrations in chronic alcoholic rats are shown in the figure below. Liver and blood triglyceride concentrations were found to be significantly highest in the Control group, and the concentration in the HT group was significantly lower than the Control group and similar to that of the Silymarin group. The PHT group was found to have significantly lower liver and blood triglyceride concentrations than the HT and Silymarin groups, and the DHD group had significantly lower concentrations than the PHT group and similar to the Normal group. Consumption of Hovenia dulcis Thunb fruit fermented vinegar beverage is believed to inhibit the production of triglycerides in the liver and blood.

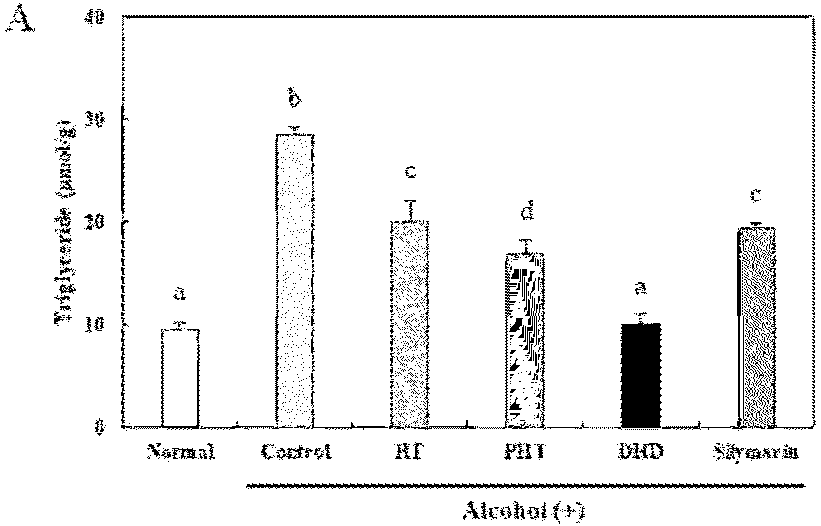

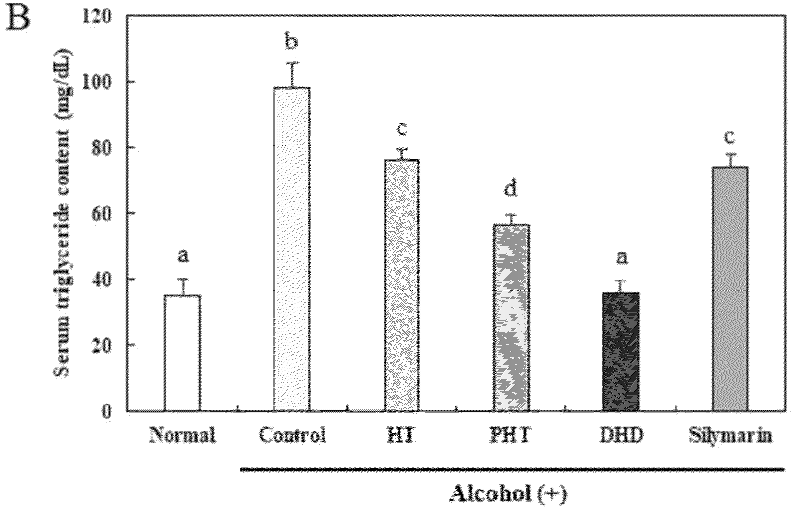

**Fig. Effects DHD on (A) triglyceride content in liver and (B) triglyceride content in serum in chronic ethanol-administered rats.** Data values were expressed as mean ± S.E. (n=6). Data values with different superscripts indicate significant difference (p 003c# 0.05) by Duncan's multiple range tests.

### H) Changes in blood total cholesterol, LDL-cholesterol, and HDL-cholesterol concentrations in a chronic alcoholic rat model

The results of blood total cholesterol, LDL-cholesterol, and HDL-cholesterol concentrations in chronic ethanol-administered rats are shown in the figure below. Total cholesterol concentration showed no significant difference in the HT and Silymarin groups compared to the Control group, and significantly decreased in the PHT group compared to the HT group. The DHD group showed a significantly lower total cholesterol concentration than the PHT group and a similar level to the Normal group. The concentration of LDL-cholesterol in the blood was significantly highest in the Control group, and the HT and Silymarin groups showed significantly lower concentrations than the Control group. The PHT group showed a significantly lower concentration than the HT group, and the DHD group showed a significantly lower concentration than the PHT group. In particular, the DHD group was confirmed to have no significant difference from the Normal group. In the case of HDL-cholesterol concentration, the control group showed the lowest concentration, and the HT group and Silymarin group showed no significant difference compared to the control group. There was no significant difference in the PHT group compared to the HT group and the Silymarin group, but the HDL-cholesterol concentration was confirmed to be increased and showed a similar concentration to the Normal group. The DHD group showed a significantly higher concentration than the Normal group. Consumption of Hovenia dulcis Thunb fruit fermented vinegar beverage was confirmed to lower total and LDL-cholesterol concentrations and increase HDL-cholesterol concentrations.

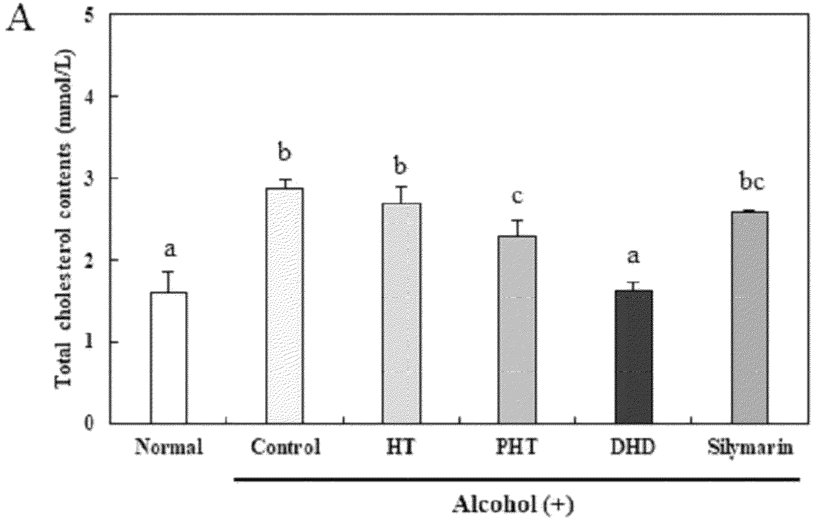

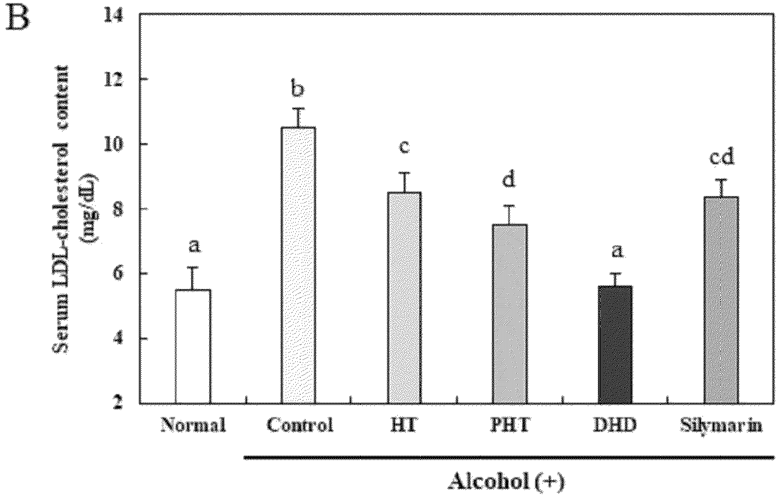

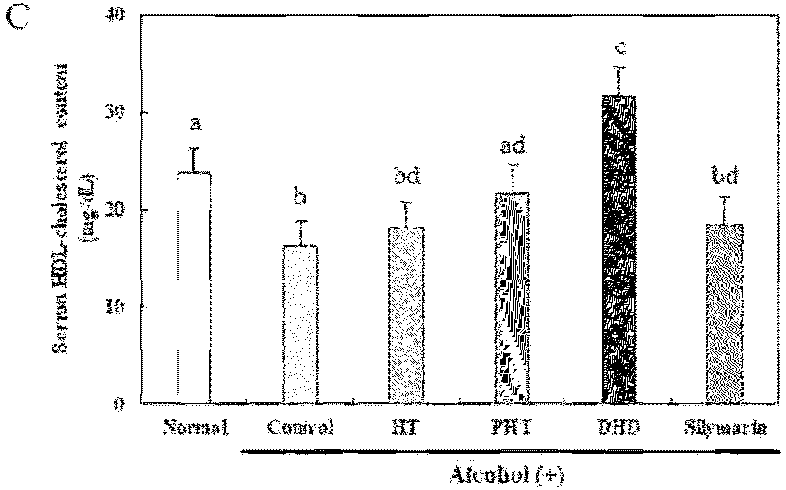

**Fig. Effects of DHD on (A) total cholesterol, (B) LDL-cholesterol and (C) HDL-cholesterol content in serum in chronic ethanol-administered rats.** Data values were expressed as mean ± S.E. (n=6). Data values with different superscripts indicate significant difference (p 003c# 0.05) by Duncan's multiple range tests.

### I) Morphological and H0026#E staining changes in liver tissue in a chronic alcoholic rat model

The morphological and H0026#E staining results of liver tissue in chronic ethanol-administered rats are as follows. When fatty liver is formed due to alcohol, the color of the liver changes from red-brown to yellow-brown. In the Control group, the color changed to yellow-brown due to the formation of fatty liver. The liver of the PHT group appeared to have a reddish-brown color compared to the Control and HT groups, and the liver of the DHD group had a dark reddish-brown color similar to that of the Normal group. As a result of H0026#E staining, many fat droplets appeared in the Control group. The HT group was confirmed to have fewer fat droplets than the Control group, and the PHT group appears to have fewer fat droplets than the HT group. The DHD group appeared to have almost no fat droplets and showed a similar shape to the Normal group. Consumption of Hovenia dulcis Thunb fruit fermented vinegar beverage is believed to suppress alcoholic fatty liver disease.

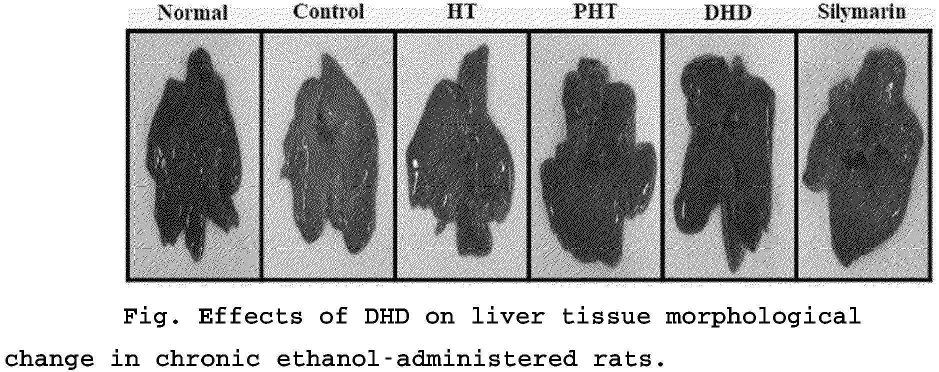

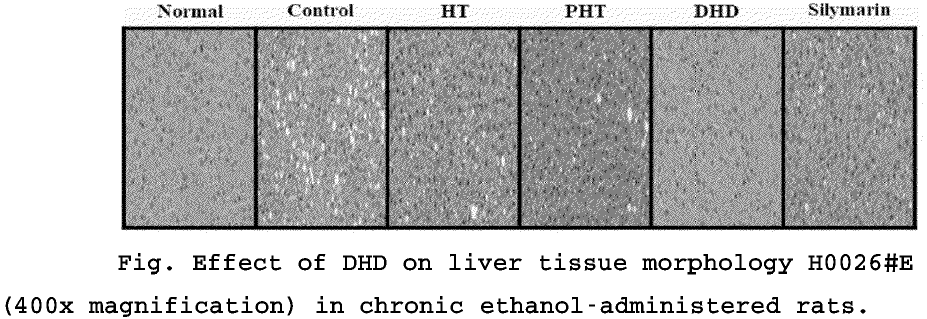

## Claims

1. A method for preparing Hovenia dulcis Thunb fruit fermented vinegar having hangover relief and liver protection effects, in which Hovenia dulcis Thunb fruits concentrate is made using Hovenia dulcis Thunb fruits, and after adjusting it to 16°Brix, 10% (v/v) of seed mash was inoculated, alcohol fermentation was performed at a temperature of 28 to 32°C, and the filtered alcohol fermentation filtrate is used as a substrate for acetic acid fermentation,
wherein the acetic acid fermentation is performed by adding alcohol fermentation liquid from Hovenia dulcis Thunb fruits, inoculating 10% (v/v) of vinegar starter, and fermenting at a temperature of 28 to 32°C and 120 to 180 rpm.

2. The method for preparing Hovenia dulcis Thunb fruit fermented vinegar having hangover relief and liver protection effects of claim 1, wherein the Hovenia dulcis Thunb fruits concentrate is made by adding water to washed Hovenia dulcis Thunb fruits in an amount 5 to 10 times the weight of the fruits, heating them, and extracting them with hot water.

3. The method for preparing Hovenia dulcis Thunb fruit fermented vinegar having hangover relief and liver protection effects of claim 1 or claim 2, wherein the alcohol fermentation is performed by subculturing the strain Saccharomyces cerevisiae (KCCM 11306) on YM plate medium and then culturing it in YM liquid medium for 48 hours.

4. The method for preparing Hovenia dulcis Thunb fruit fermented vinegar having hangover relief and liver protection effects of claim 1 or claim 2, wherein the acetic acid fermentation is performed by culturing the strain Acetobacter aceti (KCCM 12654) at 30°C for 48 hours in a liquid medium containing 2 to 3 parts by weight of mannitol, 0.1 to 0.5 parts by weight of peptone, and 0.3 to 0.8 parts by weight of yeast extract in 100 parts by weight of water.

5. A beverage composition using Hovenia dulcis Thunb fruit fermented vinegar including 0.5 to 7 parts by weight of Hovenia dulcis Thunb fruit fermented vinegar, 1 to 3 parts by weight of Hovenia dulcis concentrate, 10 to 15 parts by weight of high fructose corn syrup, 1 to 5 parts by weight of fructooligosaccharide, 0.5 to 1 parts by weight of apple concentrate, 0.1 to 0.2 parts by weight of anhydrous citric acid, 0.1 parts by weight of yeast extract powder, 0.01 to 0.03 parts by weight of Complex Scutellaria baicalensis extract, 1 to 2 parts by weight of taurine, 0.01 to 0.10 parts by weight of milk thistle extract powder, and 0.01 to 0.05 parts by weight of curcumin in 100 parts by weight of purified water.
